Europäisches Patentamt

European Patent Office

Office européen des brevets

(19)

(11) Veröffentlichungsnummer: **0 041 111**
**B1**

(12) **EUROPÄISCHE PATENTSCHRIFT**

(45) Veröffentlichungstag der Patentschrift:
28.09.83

(21) Anmeldenummer: 81102325.8

(22) Anmeldetag: 27.03.81

(51) Int. Cl.³: **A 61 F 1/00**

(54) Sehnen- und/oder Bänderersatz.

(30) Priorität: 03.06.80 CH 4278/80

(43) Veröffentlichungstag der Anmeldung:
09.12.81 Patentblatt 81/49

(45) Bekanntmachung des Hinweises auf die Patenterteilung:
28.09.83 Patentblatt 83/39

(84) Benannte Vertragsstaaten:
AT DE FR GB IT NL

(56) Entgegenhaltungen:
DE-A-2 513 284
FR-A-2 135 825
FR-A-2 213 761
FR-A-2 395 012
US-A-3 805 300
US-A-3 973 277
US-A-4 187 558

(73) Patentinhaber: **GEBRÜDER SULZER
AKTIENGESELLSCHAFT, Zürcherstrasse 9,
CH-8401 Winterthur (CH)**

(72) Erfinder: **Müller, Arnold, Prof. Dr. med. vet., Hodleten,
CH-8163 Bachs (CH)**
Erfinder: **Bröckel, Gerhard, Prof. Dr.-Ing.,
Hochgrütstrasse 4, CH-8472 Seuzach (CH)**

(74) Vertreter: **Dipl.-Ing. H. Marsch Dipl.-Ing. K. Sparing
Dipl.-Phys.Dr. W.H. Röhl Patentanwälte,
Rethelstrasse 123, D-4000 Düsseldorf (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Sehnen- und/oder Bänderersatz

Die Erfindung betrifft einen Sehnen- und/oder Bänderersatz zur Implantation in tierische oder menschliche Körper, bestehend aus einem Band einschließlich einer Anzahl, im wesentlichen in Längsrichtung des Bandes verlaufender Fäden, die von einer relativ zu ihnen beweglichen Hülle umgeben sind.

Der Begriff »Faden« soll im vorliegenden Fall alle linienförmigen Gebilde aus natürlichen Garnen, Chemiefasern, Metallen, Kohlenstoff und gegebenenfalls keramischen Werkstoffen und Mineralien umfassen. Die Auswahl der als Faden verwendeten Stoffe erfolgt — wie auch bei anderen Körperimplantanten — in erster Linie aufgrund biologisch-medizinischer Brauchbarkeit und weiterhin aufgrund der mechanischen Eigenschaften, wie Festigkeit und/oder Elastizität.

Bekanntlich setzen sich natürliche Bänder und Sehnen aus einer Vielzahl von Einzel- oder Sehnenfasern zusammen, von denen jede von einem bestimmten Ursprungsort zu einem exakt entsprechenden Ansatzpunkt zieht. Bei einer Bewegung behalten die entsprechenden Insertionsflächen ihren mittleren Abstand bei und verändern ihre Neigung zueinander; das bedingt eine gewisse Verschieblichkeit der Fasern gegeneinander. Da diejenigen Fasern, die an den entferntesten Punkten der einen Ansatzfläche ihren Ursprung haben, an den nächstliegenden Punkten der anderen ansetzen, hat das zur Folge, daß der Fasernverband in seinem Verlauf eine Drehung um die Längsachse beschreibt.

Dieser schwierige, jedoch sehr widerstandsfähige, biologische Aufbau läßt sich mit technischen Mitteln nicht in gleicher Weise nachahmen. Eine gute Annäherung daran sollte sich jedoch ergeben, wenn das künstliche, aus alloplastischen Werkstoffen gefertigte Ersatzband einen runden Aufbau mit einer vom Kern nach außen zunehmenden Elastizität hat; dabei sollte aber das künstliche Band in Längsrichtung unelastisch und nicht dehnbar sein. Mit einer derartigen Konstruktion wird erreicht, daß der Kern immer eine Zugbelastung erfährt, während der Mantel beim Abbiegen des von dem Band überbrückten Gelenkes auf Zug und Druck beansprucht, also »gestreckt« und »gestaucht« wird.

Neben dem Ersatz von Sehnen und Bändern aus körpereigenem Gewebe sind bereits eine Vielzahl alloplastischer Stoffe, wie Stahldrähte, Kunststoff-, Seiden- oder Kohlenstoff-Fäden, in der Form von Tauen oder Geweben als Ersatz für zerrissene Bänder eingesetzt worden (DE-A-2 836 921).

Die FR-A-2 213 761 betrifft eine künstliche Sehne, bei der mehrere Kunststoff-Fäden in einer Umhüllung zusammengefaßt sind, ohne daß dadurch ein Kern und ein Mantel mit erhöhter Elastizität entsteht; darüber hinaus werden die Fäden bevorzugt mit der Umhüllung durch Kleben oder Nähen verbunden, so daß beide Komponenten relativ zueinander fixiert sind.

Eine von der Umhüllung lose umschlossene Sehne, die in einem geflochtenen Bündel aus mit pyrolitischem Kohlenstoff beschichteten Kunststoff-Fäden besteht, offenbart die FR-A-2 395 012. Auch bei dieser Konstruktion ist kein Kern mit einem ihn umgebenden Mantel vorhanden, sondern das geflochtene Bündel hat über seinen ganzen Querschnitt die gleichen Eigenschaften.

Das künstliche Band nach der US-A-3 805 300 besteht aus einem Streifen aus Kunststoff, in den eine geflochtene Armierung einvulkanisiert ist; fensterartigen Öffnungen in den Kunststoff-Streifen verleihen diesem künstlichen Band eine gewisse Dehnbarkeit in Längsrichtung. Den beschriebenen Anforderungen an eine künstliche Sehne kann dieses künstliche Band daher nicht genügen.

Aufgabe der Erfindung ist es somit, ein in Längsrichtung nicht dehnbares, weitgehend unelastisches künstliches Band zu schaffen, bei dem der Abrieb auf ein Minimum reduziert ist und das darüber hinaus die erwähnte Forderung eines runden Aufbaues mit einer nach außen zunehmenden Elastizität möglichst weitgehend erfüllt.

Diese Aufgabe wird erfindungsgemäß dadurch gelöst, daß das Band aus mindestens einem runden Strang besteht, in dem ein Kernfaden von mindestens einem Kranz aus Mantelfäden geringeren Durchmessers umgeben ist, daß ferner jeder einzelne Faden in einem relativ zu ihm beweglichen Schlauch verläuft, und daß schließlich alle Fäden des Stranges von der Stranghülle umschlossen sind.

Durch die Einbettung jeden Fadens in einen Schlauch, der vorzugsweise aus einem textilen Geflecht aus Naturseide, aber auch aus einem Kunststoff-Schlauch — wie er beispielsweise als Isolierschlauch aus der Elektrotechnik bekannt ist — bestehen kann, wird Abrieb an den einzelnen Kern- und Mantelfäden vermieden; durch geeignete Wahl des Materials ist es weiterhin möglich, die Bänder in Längsrichtung für die auftretende Belastung unelastisch auszubilden. Beispielsweise können die Fäden vorteilhafterweise in einem Draht aus einem Metall oder einer Metall-Legierung mit Durchmessern von 0,1 bis 1 mm für die Kern- und von 0,05 bis 0,5 mm für die Mantelfäden bestehen.

Der Aufbau aus einzelnen, relativ dünnen Fäden gewährleistet dabei trotz der unelastischen Fäden in Längsrichtung eine große Biegeelastizität des Bandes und die besonders bei Streßbelastungen notwendige Dehnbarkeit vorwiegend in Richtung senkrecht zur Längsachse.

Ein rotationssymmetrischer, konzentrischer Aufbau des Stranges aus einem Kernfaden und diesen umgebenden Mantelfäden, die geringere Durchmesser als der Kernfaden haben, sichert die geforderte nach außen zunehmende Elastizität.

Eine Möglichkeit für die Verwirklichung von stärkeren Bändern mit nach außen zunehmender Elastizität besteht darin, das Band aus einem Strang mit mehr als einem Mantel um den Kern zu fertigen und dabei die Durchmesser der Mantelfäden vom Kern nach außen von Kranz zu Kranz abnehmen zu lassen; dabei ist es zusätzlich möglich, zwischen den einzelnen Kränzen der Mantelfäden Hüllen vorzusehen.

Die geforderten Eigenschaften können bei der Schaffung von Bändern für stärkere Belastungen aber auch erreicht werden, wenn zum Aufbau stärkerer Bänder mehrere Stränge für ein Kabel bilden, wobei die einzelnen Stränge ebenfalls nach dem Prinzip eines, von mindestens einem Mantel umgebenden Kerns angeordnet und in einer gemeinsamen Kabelhülle zusammengefaßt sind; dabei ist es zweckmäßig, gegebenenfalls in dem oder den Kränzen des Mantels eine unterschiedliche Anzahl, beispielsweise 3 bis 6 Stänge, unterzubringen. Es ist jedoch auch möglich, unterschiedliche Stränge für den Aufbau von Kernstrang und Mantelsträngen des Kabels zu verwenden. Beispielsweise kann als Kernstrang ein Strang eingesetzt werden, der selbst mehrere Kränze von Mantelfäden um seinen Kern herum besitzt, während als Mantelstränge mit nur einem Kranz mit Mantelfäden ausgerüstete Stränge dienen.

In gleicher Weise können bei Bändern, die extreme Belastungen ausgesetzt sind, wiederum mehrere Kabel in dem geschilderten Kern/Mantel-Aufbau zu einem Band zusammengesetzt werden, wobei die nach außen zunehmende Elastizität z. B. dadurch erreicht werden kann, daß als Kern ein Kabel mit einer hohen Anzahl von Strängen in seinem Mantel und als Mantel Kabel mit weniger Strängen in ihren Mänteln benutzt werden.

In Anlehnung an die eingangs erwähnte Drehung um die Längsachse, die der natürliche Fasernverband in seinem Verlauf ausführt, kann es schließlich vorteilhaft sein, wenn die Mantelfäden und/oder Mantelstränge bzw. -Kabel schraubenförmig um ihren Kern gewunden sind.

Im folgenden wird die Erfindung anhand von Ausführungsbeispielen im Zusammenhang mit der Zeichnung näher erläutert.

Fig. 1 zeigt den Aufbau eines Stranges im Querschnitt;

Fig. 2 ist, ebenfalls im Querschnitt, eine zweite Ausführungsform für einen Strang;

Fig. 3a bis 3d geben, wiederum im Querschnitt, die Zusammenfassung von Strängen zu Kabeln wieder, wobei bei den einzelnen Kabeln in den den Kern umhüllenden Mänteln eine unterschiedliche Anzahl Stränge untergebracht ist;

Fig. 4 stellt im Querschnitt ein Band dar, zu dem mehrere Kabel vereinigt sind;

Fig. 5 ist eine Aufsicht auf eine Ausführungsform eines Verankerungselementes für die Befestigung eines Bandes an einem Knochen, während

Fig. 6 der Schnitt VI-VI von Fig. 5 ist.

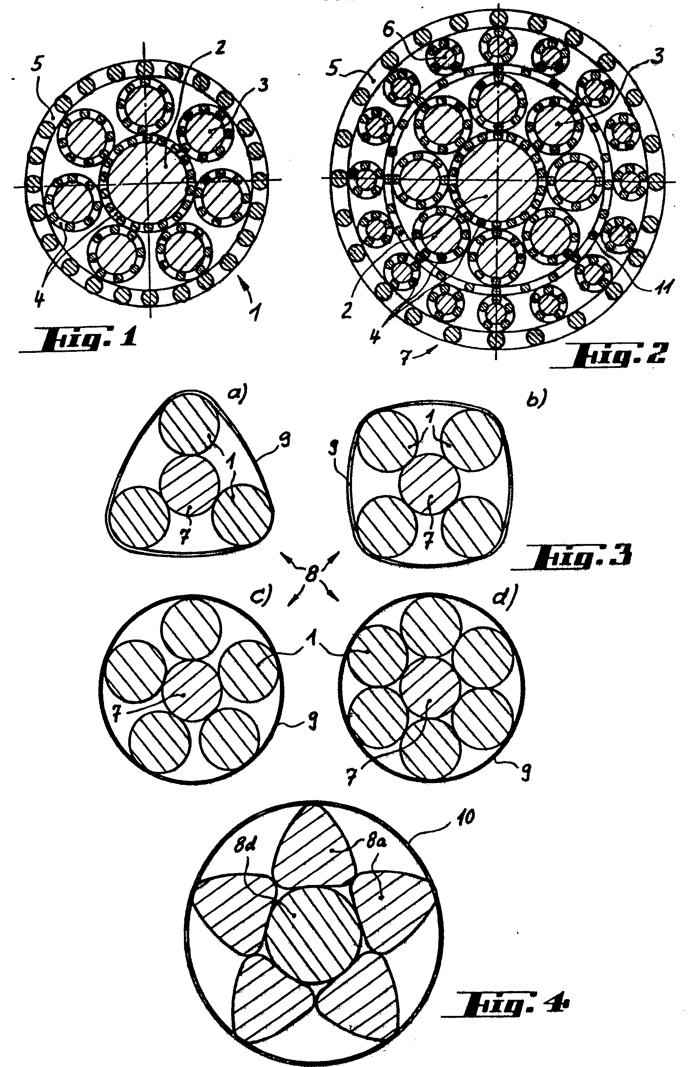

Bei der Ausführungsform nach Fig. 1 besitzt ein in seiner Gesamtheit mit 1 bezeichneter Strang einen Kernfaden 2, der in diesem Fall in einem Metalldraht aus einer in der Implantattechnik üblichen Metall-Legierung, beispielsweise einer CoNiCrMoTi-Schmiedelegierung, besteht. Um den Kernfaden 2 herum, sind in einem Kranz Mantelfäden 3 aus der gleichen Legierung konzentrisch und rotationssymmetrisch angeordnet. Diese Mantelfäden 3 sind — was aus der Zeichnung nicht ersichtlich ist — mit Vorteil leicht seilartig verwunden, d. h. die Mantelfäden 3 verlaufen in Längsrichtung schraubenförmig um den Kernfaden 2 herum.

Jeder einzelne Faden 2 bzw. 3 steckt bei der vorliegenden Ausführungsform in einem textilen Schlauch 4, durch den, wie bereits erwähnt, ein Abrieb an den die Belastungen aufnehmenden Drähten bzw. Fäden 2, 3 bei den zwischen den einzelnen »Fasern« des Bandes oder des Stranges auftretenden relativen Längsverschiebungen verhindert oder zumindest vermindert wird.

Die in ihren Schläuchen 4 verlaufenden Kern- und Mantelfäden 2 bzw. 3 sind als Ganzes von einer Stranghülle 5 umschlossen, durch die ihr mechanischer Zusammenhalt und die relative Lage der Mantelfäden 3 auf dem Umfang des Kranzes gewährleistet werden.

Die Ausführungsform eines Stranges 7 nach Fig. 2 unterscheidet sich von derjenigen nach Fig. 1 lediglich dadurch, daß der Kernfaden 2 von zwei Kränzen aus Mantelfäden 3 bzw. 6 umgeben ist, wobei der Durchmesser der Mantelfäden 3 größer ist als derjenige der Mantelfäden 6 des äußeren Kranzes. Der Zusammenhalt des Stranges 7 ist auch hier durch eine Stranghülle 5 sichergestellt, wobei zur geometrischen Fixierung der Mantelfäden in den einzelnen Kränzen zusätzliche Hüllen 11 zwischen den Kränzen der Mantelfäden 3 bzw. 6 vorgesehen sein können; diese zusätzlichen textilen Hüllen erleichtern und vereinfachen vor allem das erwähnte Verseilen der Mantelfäden um den Kern herum.

Die Durchmesser des Kernfadens 2 können beispielsweise zwischen 0,1 und 1 mm betragen, während die Mantelfäden 3 bzw. 6 Durchmesser zwischen 0,05 und 0,5 mm haben. Die Abstimmungen zwischen dem Durchmesser des Kernfadens 2 und denjenigen der ihm im Einzelfall zugeordneten Mantelfäden 3 bzw. 6 erfolgt dabei so, daß die Durchmesser der Fäden vom Kern nach außen von Kranz zu Kranz (Fig. 2) abnehmen, um die Elastizität des Stranges des daraus gebildeten Bandes gemäß den eingangs erwähnten Forderungen von innen nach außen zu steigern.

Die Schläuche 4 und die Stranghülle 5 sind beispielsweise ein Hohlgeflecht, wobei die der deutlichen Darstellung wegen mit großen Abständen gezeichneten einzelnen Flechtfäden auch so dicht wie möglich gepackt sein können; die Dichte des Geflechtes kann dabei im Kern wiederum größer sein als bei den Mantelfäden und bei der Stranghülle. Als bevorzugtes Material für die Schläuche 4 oder Stranghülle 5 dient Naturseide; fabrikatorisch einfacher ist es, die einzelnen Fäden 2 und 3 — ähnlich wie für Isolationen elektrischer Leitungen bekannt — mit Kunststoff zu ummanteln, was beispielsweise durch Tauchen des Fadens in ein Bad aus einem geeigneten Kunststoff erfolgen kann. Ebenso können die Stranghüllen aus derartigen Kunststoffmänteln bestehen.

Obwohl bereits ein Strang 1 bzw. 7 als Bänder- oder Sehnenersatz direkt zu verwenden ist, ist es auch möglich, mehrere Stränge 1 bzw. 7 für den Aufbau eines Bandes mit höherer Belastungsfähigkeit zu benutzen. Wie in Fig. 3 gezeigt, bildet dabei beispielsweise ein Strang 7 den Kernstrang eines Kabels 8, den Stränge 1 als Mantelstränge konzentrisch und rotationssymmetrisch umgeben, wobei in dem Mantelkranz drei, vier oder mehr — je nach Durchmesser-Unterschied zwischen den Strängen 7 und 1, beispielsweise bis zu acht — Stränge angeordnet sein können. Die geforderte nach außen zunehmende Elastizität ist dabei beispielsweise durch die Verwendung eines steiferen Stranges 7 als Kernstrang und von elastischeren Strängen 1 als Mantelstränge gewährleistet, wobei die Elastizität der äußeren Bereiche durch eine verschieden große Anzahl der in einem Kranz vorgesehenen Stränge 1 in gewissem Umfang variiert werden kann. Je weniger Stränge 1 dabei in einem Kranz angeordnet werden, um so größer ist dabei außen die Elastizität. In Fig. 3 sinkt dabei die Elastizität außen stufenweise von Fig. 3a—3d.

Jedes Kabel 8 ist dabei durch eine Kabelhülle 9 aus einem Naturseide-Geflecht zu einer Einheit zusammengefaßt.

Selbstverständlich können — wie bei den Strängen — auch Kabel aus einem Kernstrang und zwei oder mehreren Kränzen Mantelsträngen zusammengefügt werden.

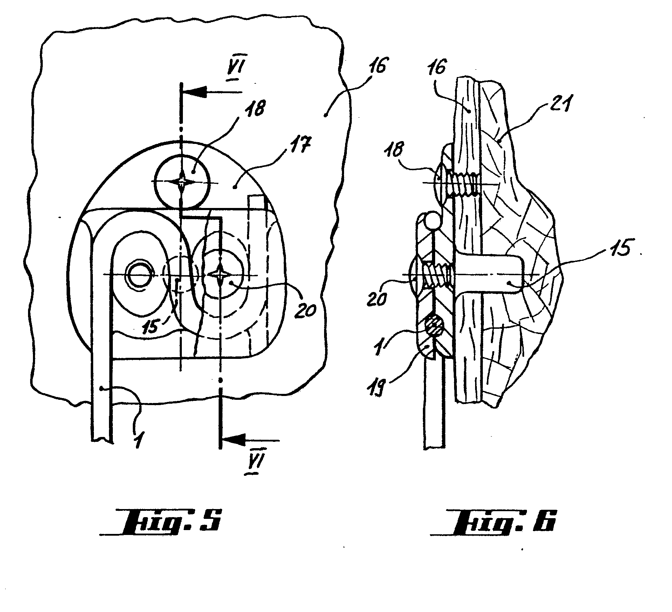

Für Bänder, die extremen Belastungen ausgesetzt sind, lassen sich wiederum mehrere Kabel 8 zu einem Band vereinigen, wobei als Kernkabel beispielsweise ein Kabel 8d nach Fig. 3d verwendet wird, während die Mantelkabel aus Kabeln 8a nach Fig. 3a gebildet sind, wie dies Fig. 4 zeigt. Zusammengehalten wird auch dieses Band durch ein Naturseide-Geflecht als textile Umhüllung 10.

Als Beispiel einer Verankerung eines Bandendes am Knochen ist nach Fig. 5 und Fig. 6 eine Platte 17 vorgesehen, die mit einem Zapfen 15 durch die kortikale Schicht 16 in die Spongiosa 21 eingelassen und durch eine oder mehrere Schrauben 18 auf der kortikalen Schicht 16 befestigt ist. Auf diese Platte 17 ist ein Deckel 19 gesetzt und durch Schrauben 20 gehalten. Die Platte 17 und der Deckel 19 bilden zusammen zwei pollerartige Pfosten, wie sie zum Vertauen von Schiffen an Landungsstegen bekannt sind.

Wie Fig. 5 zeigt, wird ein zu befestigendes Band 1 einfach um diese Pfosten geschlungen und durch Anziehen der den Deckel 19 auf die Platte 17 ziehenden Schrauben 20 festgeklemmt.

Selbstverständlich ist die vorliegende Erfindung nicht auf die gezeigten Ausführungsbeispiele beschränkt, insbesondere nicht hinsichtlich der ausgewählten Materialien und hinsichtlich der skizzierten Verankerung des künstlichen Bandes am Knochen.

## Patentansprüche

1. Sehnen- und/oder Bänderersatz zur Implantation in tierische oder menschliche Körper, bestehend aus einem Band einschließlich einer Anzahl, im wesentlichen in Längsrichtung des Bandes verlaufender Fäden (2, 3), die von einer relativ zu ihnen beweglichen Hülle (5) umgeben sind, dadurch gekennzeichnet, daß das Band aus mindestens einem runden Strang (1, 7) besteht, in dem ein Kernfaden (2) von mindestens einem Kranz aus Mantelfäden (3) geringeren Durchmessers umgeben ist, daß ferner jeder einzelne Faden (2, 3) in einem relativ zu ihm beweglichen Schlauch (4) verläuft, und daß schließlich alle Fäden (2, 3) des Stranges (1, 7) von der Stranghülle (5) umschlossen sind.

2. Sehnen- und/oder Bänderersatz nach Anspruch 1, dadurch gekennzeichnet, daß zum Aufbau stärkerer Bänder mehrere Stränge (1, 7) ein Kabel (8) bilden, wobei die einzelnen Stränge ebenfalls nach dem Prinzip eines von mindestens einem Mantel umgebenen Kernes angeordnet und durch eine gemeinsame Kabelhülle (9) zusammengefaßt sind.

3. Sehnen- und/oder Bänderersatz nach Anspruch 2, dadurch gekennzeichnet, daß ein Band aus mehreren Kabeln (8) mit unterschiedlicher Anzahl von Strängen (1, 7) nach dem Prinzip eines mindestens von einem Mantel umgebenen Kerns aufgebaut und von einer gemeinsamen Umhüllung (10) umgeben ist.

4. Sehnen- und/oder Bänderersatz nach einem der Ansprüche 1 bis 3, dadurch gekennzeichnet, daß die Schläuche (4), die Stranghüllen (5), die Kabelhüllen (9) und/oder die Umhüllungen (10) aus textilen Geflechten bestehen.

5. Sehnen- und/oder Bänderersatz nach Anspruch 4, dadurch gekennzeichnet, daß das textile Material für die Schläuche (4), Stranghüllen (5), Kabelhüllen (9) und Umhüllungen (10) Naturseide ist.

6. Sehnen- und/oder Bänderersatz nach einem der vorangehenden Ansprüche, aus Strängen (7) mit mehr als einem Kranz Mantelfäden (3, 6), dadurch gekennzeichnet, daß die Durchmesser der Mantelfäden (3, 6) vom Kern (2) nach außen von Kranz zu Kranz abnehmen.

7. Sehnen- und/oder Bänderersatz nach einem der vorangehenden Ansprüche, dadurch gekennzeichnet, daß die Mantelfäden (3, 6) und/oder Mantelstränge (1) bzw. -Kabel (8a) schraubenförmig um ihren Kern (2, 7, 8d) gewunden sind.

## Claims

1. A tendon and/or ligament prosthesis for implanting in animal or human organisms, comprising a band or tape or the like inclusive of a number of filaments (2, 3) which extend substantially lenghtwise of such band or tape or the like and around which an envelope (5) movable relatively to the filaments extends, characterised in that the band or tape or the like comprises at least one circular strand (1, 7) in which a core filament (2) has around it at least one ring of smaller-diameter outer filaments (3); each individual filament (2, 3) extends in a flexible tube (4) movable relatively to the filament inside it; and the strand envelope (5) extends around all the filaments (2, 3) of the strand (1, 7).

2. A prosthesis according to claim 1, characterised in that a number of strands (1, 7) form a cable (8) to provide relatively thick bands or tapes or the like, the discrete strands also being arranged on the basis of a core around which at least one cover extends and being held together by a common cable envelope (9).

3. A prosthesis according to claim 2, characterised in that a band comprising a number of cables (8) having a different number of strands (1, 7) is devised on the basis of a core around which at least one cover extends, a common envelope (10) extending around the multi-cable band or tape or the like.

4. A prosthesis according to any of claims 1 to 3, characterised in that flexible tubes (4), strand envelopes (5), cable envelopes (9) and/or common envelopes (10) are embodied by braided textile structures.

5. A prosthesis according to claim 4, characterised in that the textile material for the flexible tubes (4), strand envelopes (5), cable envelopes (9) and common envelopes (10) is natural silk.

6. A prosthesis according to any of the previous claims devised from strands (7) having more than one ring of outer yarns (3, 6), characterised in that the diameters of the outer yarns (3, 6) decrease outwardly, starting from the core (2), from ring to ring.

7. A prosthesis according to any of the previous claims, characterised in that the outer filaments (3, 6) and/or outer strands (1) or outer cables (8a) extend helically around their respective core (2, 7, 8d).

## Revendications

1. Cordes et/ou bandes de remplacement destinées à une implantation dans le corps humain ou le corps d'animaux, consistant en une bande incluant plusieurs fils (2, 3) s'étendant sensiblement dans le sens longitudinal de la bande et qui sont entourés par une enveloppe (5) relativement mobile par rapport aux fils, caractérisées en ce que la bande est constituée par au moins un boyau rond (1, 7) dans lequel un fil de noyau (2) est entouré par au moins une couronne de fils de manteau (3) de diamètre plus faible, en ce qu'en outre chaque fil individuel (2, 3) s'étend dans un flexible (4) relativement mobile par rapport au fil, et en ce que finalement tous les fils (2, 3) du boyau (1, 7) sont enfermés dans une enveloppe boyau (5).

2. Cordes et/ou bandes de remplacement selon la revendication 1, caractérisées en ce que pour constituer des bandes plus résistantes, plusieurs boyaux (1, 7) forment un câble (8), les différents boyaux étant disposés également selon le principe d'un noyau entouré par au moins un manteau et étant rassemblés par une enveloppe commune (9) de câbles.

3. Cordes et/ou bandes de remplacement selon la revendication 2, caractérisées en ce qu'une bande en plusieurs câbles (8) ayant un nombre différent de boyaux (1, 7) est constituée selon le principe d'un noyau entouré par au moins un manteau et est entourée par un enveloppement commun (10).

4. Cordes et/ou bandes de remplacement selon l'une des revendications 1 à 3, caractérisées en ce que les flexibles (4), les enveloppes boyaux (5), les enveloppes de câbles (9) et/ou les enveloppements (10) sont constitués par des tresses textiles.

5. Cordes et/ou bandes de remplacement selon la revendication 4, caractérisées en ce que la matière textile destinée aux flexibles (4), enveloppes boyau (5), enveloppes de câbles (9) et enveloppements (10) est de la soie naturelle.

6. Cordes et/ou bandes de remplacement selon l'une des revendications précédentes, en boyaux (7) ayant des fils de manteau (3, 6) formés de plus d'une couronne, caractérisées en ce que les diamètres des fils de manteau (3, 6) diminuent du noyau (2) vers l'extérieur, de couronne à couronne.

7. Cordes et/ou bandes de remplacement selon l'une des revendications précédentes, caractérisées en ce que les fils de manteau (3, 6) et/ou les boyaux (1) ou câbles (8a) de manteau sont enroulés hélicoïdalement autour de leur noyau (2, 7, 8d).

0 041 111

**Fig. 1**

**Fig. 2**

a)

b)

**Fig. 3**

c)

d)

**Fig. 4**

**Fig. 5**

**Fig. 6**